# EUROPÄISCHE PATENTSCHRIFT

(11) **EP 2 823 790 B1**
(45) Veröffentlichungstag und Bekanntmachung des Hinweises auf die Patenterteilung: **03.05.2017**
(21) Anmeldenummer: 14174481.3
(22) Anmeldetag: 26.06.2014
(51) Int. Cl.: A61F 2/30, A61F 2/34, A61F 2/36, A61F 2/38, A61F 2/40, A61F 2/42

(54) **Zweiteiliger artikulierender Gelenkspacer und Verfahren zu dessen Herstellung**
Two-part articulating joint spacer and method for producing the same
Espaceur d'articulation à articulation bilatérale et son procédé de fabrication

(30) Priorität: 08.07.2013 DE 102013011296
(43) Veröffentlichungstag der Anmeldung: 14.01.2015
(73) Patentinhaber: Heraeus Medical GmbH, 61273 Wehrheim (DE)
(72) Erfinder: Kim, Hieng, 61130 Nidderau (DE)
(74) Vertreter: Sterzel, Roland

(56) Entgegenhaltungen:
- DE-B4-102007 004 968
- US-A- 5 123 927
- US-A1- 2005 107 885
- US-B1- 6 361 731

## Beschreibung

Die Erfindung betrifft einen artikulierenden Gelenkspacer zum temporären Ersetzen eines Gelenks, wobei der Gelenkspacer zwei Spacerteile aufweist, die jeweils eine Gleitfläche aufweisen, auf der die Spacerteile im beim Patienten eingesetzten Zustand beweglich aneinander anliegen und aufeinander abrollen, Ferner betrifft die Erfindung ein Verfahren zur Herstellung eines zweiteiligen artikulierenden Gelenkspacers.

Gelenkendoprothesen haben gegenwärtig eine Standzeit von mehreren Jahren. Es gibt jedoch unerwünschte Lockerungen der Gelenkendoprothesen, die vor dem Erreichen der üblichen Standzeiten auftreten können. Es wird dabei die septische und die aseptische Lockerung unterschieden. Bei der aseptischen Lockerung lassen sich bisher keine mikrobiellen Keime nachweisen. Die Ursachen der aseptischen Lockerungen können vielfältig sein. Häufig sind aseptische Lockerungen auf Abrieb an den Gleitflächen der Gelenkendoprothesen zurückzuführen. Bei der septischen Lockerung wird der Lockerungsprozess durch mikrobielle Keime hervorgerufen. Man unterscheidet dabei in Abhängigkeit vom zeitlichen Auftreten frühe und späte Infektionen. Die septische Lockerung ist eine sehr ernste Erkrankung für den Patienten, die zusätzlich mit sehr hohen Kosten verbunden ist. Sowohl bei der aseptischen als auch der septischen Lockerung wird üblicherweise eine Revision vorgenommen. Man unterscheidet einzeitige und die zweizeitige Revisionen. Bei septischen Lockerungen werden sehr häufig zweizeitige Revisionen vorgenommen.

Bei der zweizeitigen Revision wird in einer ersten Operation (OP) die infizierte Gelenkendoprothese entfernt, es erfolgt ein Debridement des infizierten Gewebes und anschließend wird ein temporärer Platzhalter, ein so genannter Spacer, eingesetzt. Dieser Spacer füllt für einige Wochen den Raum der zuvor revidierten Endoprothese aus, bis die vorliegende Infektion abgeklungen ist. Diese Platzhalterfunktion ist sehr wichtig, um eine Atrophie der Muskulatur in dieser Zeit wirksam zu verhindern und um eine Stabilisierung der Resektionssituation zu erreichen. Man unterscheidet dann nichtartikulierende und artikulierende Spacer. Artikulierende Spacer oder Gelenkspacer bilden die Gelenkfunktion nach und erlauben eine gewisse Beweglichkeit der betroffenen Gliedmaßen. Es ist dadurch möglich, die Patienten frühzeitig zu mobilisieren. Artikulierende Gelenkspacer stellen heute den Stand der Technik dar. Der Spacer wird in einer zweiten OP entfernt, es wird nochmals debridiert und dann wird eine zementierte oder auch zementfreie Revisions-Gelenkendoprothese implantiert.

Es gibt auf dem Markt mit Antibiotika ausgerüstete Spacer zum temporären Ersatz von Knie-, Hüft- und Schultergelenkendoprothesen. Solche Spacer sind beispielsweise aus der US 2010/042214 A1 und der US 2011/015754 A1 bekannt. Die dort beschriebenen Spacer enthalten Hohlräume, aus denen die Antibiotika freigesetzt werden. Nachteilig ist hieran die ungleichmäßige Wirkstofffreisetzung. Alternativ dazu wurden beispielsweise mit der DE 10 2007 004 968 B4 und der WO 2011 086788 A1 auch Knochenzemente vorgeschlagen, aus denen die Antibiotika löslich sind. Bei der Verwendung solcher Knochenzemente zur Herstellung eines Spacers werden die Antibiotika direkt am Ort der Infektion über einen längeren Zeitraum freigegeben und müssen daher aus dem Material des Spacers lösbar sein. Ein solcher Spacer aus Knochenzement wird beispielsweise in der Patentanmeldung US 2012/0261546 A1 vorgeschlagen. Die US 6 361 731 B1 offenbart eine Spacerform, die mit einem Knochenzement befüllt werden kann, um einen Spacer zu erzeugen, sowie einen artikulierenden Gelenkspacer gemäss dem Oberbegriff des Anspruchs 1.

Spacer werden häufig vom Chirurgen aus konventionellen Polymethylmethacrylat-Knochenzementen (PMMA-Knochenzementen) unter Nutzung geeigneter Gießformen selbst hergestellt. Dabei werden dem PMMA-Knochenzementpulver vor der Spacer-Herstellung ein oder mehrere Antibiotika zugemischt. Zusätzlich kann auch Zirkonoxidpulver als Röntgenopaker eingebracht oder aufgebracht werden, das als Kontrastmittel für Röntgenuntersuchungen dient, wie beispielsweise mit der DE 103 40 800 A1 oder der FR 2 821 751 A1 vorgeschlagen. Nachteilig an solchen Spacern ist, dass sich die Antibiotika haltigen Spacer mit dem Zirkonoxidpulver an den Gleitflächen abnutzen. Dies hat den Nachteil, dass die Gleitflächen bei einer mechanischen Belastung beeinträchtigt werden können. Insbesondere bei artikulierenden Spacern, bei denen die Gleitflächen der Spacer aufeinander abrollen, kann es zu einem Abrieb kommen. Ein solcher Abrieb kann zu Entzündungen führen, die sich Nachteilig auf die Heilung auswirken.

Die Aufgabe der Erfindung besteht also darin, die Nachteile des Stands der Technik zu überwinden. Insbesondere soll ein erfindungsgemäßer Spacer und ein erfindungsgemäßes Verfahren zu seiner Herstellung eine Möglichkeit bereitstellen, den Abrieb zu verhindern und auf einfache Weise eine möglichst ebene, stabile und robuste Artikulationsoberfläche zu erzeugen, die eine problemfreie Bewegung des erzeugten Spacers ermöglicht, ohne dass es zu einer Verschlechterung der Beweglichkeit des Gelenks, zu einer Beeinträchtigung der Heilung oder zu Schmerzen beim Patienten aufgrund eines Abriebs an den artikulierenden Gleitflächen des Spacers kommt. Die Vorrichtung und das Verfahren sollen möglichst universell einsetzbar sein.

Die Aufgaben der Erfindung werden gelöst durch einen artikulierenden Gelenkspacer zum temporären Ersetzen eines Gelenks, wobei der Gelenkspacer zwei Spacerteile aufweist, die jeweils eine Gleitfläche aufweisen, auf der die Spacerteile im beim Patienten eingesetzten Zustand beweglich aneinander anliegen und aufeinander abrollen, wobei die Gleitflächen durch einen abriebarmen ersten Knochenzement gebildet sind und die restlichen Spacerteile zumindest teilweise aus einem im Vergleich zum ersten Knochenzement unterschiedlichen zweiten Knochenzement gebildet sind, der wenigstens ein in Wasser lösliches Antibiotikum enthält, wobei der erste Knochenzement im Bereich der Gleitflächen mit einer Dicke von zumindest 1 mm auf dem zweiten Knochenzement angeordnet ist.

Bei beiden Knochenzementen handelt es sich bevorzugt um Polymethylmethacrylat-Knochenzemente.

Dabei kann vorgesehen sein, dass der erste Knochenzement im Bereich der Gleitflächen mit einer Dicke zwischen 2 mm und 15 mm angeordnet ist, besonders bevorzugt mit einer Dicke zwischen 6 mm und 11 mm angeordnet ist.

Diese Dicken sind ausreichend, um eine gute Stabilität der durch den ersten Knochenzement gebildeten Gleitflächen zu gewährleisten.

Des Weiteren kann vorgesehen sein, dass der erste Knochenzement zumindest eine Verankerung aufweist, die sich aus Richtung der Gleitfläche konisch in die restlichen Spacerteile fortsetzt, insbesondere in den zweiten Knochenzement fortsetzt.

Hierdurch wird eine stabilere Verbindung der beiden Knochenzemente erreicht. Zudem kann so die Menge des verwendeten kostenaufwendigen Antibiotika haltigen Zements reduziert werden. Mit einer Weiterentwicklung der Erfindung wird vorgeschlagen, dass die restlichen Spacerteile vollständig aus dem zweiten Knochenzement bestehen und zumindest die Verbindungen des Gelenkspacers zum Knochen des Patienten aus dem zweiten Knochenzement bestehen, bevorzugt auch die von der Gleitfläche beabstandeten Bereiche der Gelenkköpfe des Gelenkspacers aus dem zweiten Knochenzement bestehen, besonders bevorzugt die von der Gleitfläche zumindest 1 mm beabstandeten Bereiche der Gelenkköpfe des Gelenkspacers aus dem zweiten Knochenzement bestehen.

Hierdurch wird sichergestellt, dass der Aufbau des Gelenkspacers möglichst einfach ist. Zudem können so möglichst große Teile der Oberfläche, die nicht zur beanspruchten Gleitfläche der Spacerteile gehören, zum Freisetzen von Antibiotika genutzt werden. Das Antibiotikum im zweiten Knochenzement wird vorzugsweise ausgewählt aus den Gruppen der Aminoglykosid-Antibiotika, der Glykopeptid-Antibiotika, der Lincosamid-Antibiotika, der Chinolon-Antibiotika, der Oxazolidinon-Antibiotika, der Gyrasehemmer, der Carbapeneme, der cyclischen Lipopeptide, der Glycylcycline und der Peptidantibiotika.

Gemäß einer besonders bevorzugten Ausführungsform handelt es sich bei dem Antibiotikum um ein Mitglied, das aus der Gruppe ausgewählt ist, die aus Gentamicin, Tobramycin, Amikacin, Vancomycin, Teicoplanin, Dalbavancin, Lincosamin, Clindamycin, Moxifloxacin, Levofloxacin, Ofloxacin, Ciprofloxacin, Doripenem, Meropenem, Tigecyclin, Linezolid, Eperezolid, Ramoplanin, Metronidazol, Tinidazol, Omidazol und Colistin sowie Salzen und Estern davon besteht.

Demnach kann das wenigstens eine Antibiotikum aus der Gruppe ausgewählt sein, die aus Gentamicinsulfat, Gentamicinhydrochlorid, Amikacinsulfat, Amikacinhydrochlorid, Tobramycinsulfat, Tobramycinhydrochlorid, Clindamycinhydrochlorid, Lincosaminhydrochlorid und Moxifloxacin besteht.

Es ist erfindungsgemäß besonders bevorzugt, wenn vorgesehen ist, dass der abriebarme erste Knochenzement ein röntgenopakes Pulver mit einer Mohshärte von weniger als 8 beinhaltet, bevorzugt eine Mohshärte von weniger als 6, besonders bevorzugt eine Mohshärte von weniger als 4 beinhaltet.

Je geringer die Härte des röntgenopaken Pulvers des ersten Knochenzements ist, desto weniger tragen diese zum Abreiben der Gleitflächen bei.

Besonders bevorzugt kann vorgesehen sein, dass die Härte des röntgenopaken Pulvers im ersten Knochenzement auf die Härte des restlichen ersten Knochenzements abgestimmt ist, bevorzugt die Differenz der Mohshärte der Komponenten weniger als 2 beträgt, besonders bevorzugt weniger als 1. Wenn die Härte des röntgenopaken Pulvers auf die Härte der anderen festen Komponente, beziehungsweise der anderen festen Komponenten abgestimmt ist, wird der Abrieb der Gleitflächen deutlich reduziert.

Es kann auch vorgesehen sein, dass der zweite Knochenzement eine Mischung aus zumindest zwei Antibiotika enthält, bevorzugt ausgewählt aus Gentamicin, Vancomycin und Clindamycin.

Solche Mischungen sind besonders gut zur Behandlung der Infektionen am Gelenk geeignet.

Des Weiteren kann vorgesehen sein, dass das Innere der Spacerteile aus dem ersten Knochenzement besteht.

Durch diese Maßnahme kann die Menge des verwendeten zweiten Knochenzements verringert werden, was zum einen Kosten spart und zum anderen den unnötigen Einsatz von Antibiotika vermeidet.

Mit einer besonders bevorzugten Ausgestaltung der Erfindung wird vorgeschlagen, dass die freie Oberfläche des ersten Knochenzements mit zumindest einem Antibiotikum beschichtet ist.

Hierdurch kann auch diese Oberfläche zumindest unmittelbar nach dem Einsetzen zur Bekämpfung der Infektion beitragen.

Gemäß einer bevorzugten Ausgestaltung der Erfindung kann vorgesehen sein, dass der abriebarme erste Knochenzement ein Calciumcarbonatpulver und/oder Bariumcarbonatpulver beinhaltet.

Das Calciumcarbonatpulver und das Bariumcarbonatpulver weisen zum einen die gewünschten röntgenopaken Eigenschaften auf, so dass der Spacer bei einer Röntgenaufnahme den gewünschten Kontrast erzeugt. Gleichzeitig ist die Härte dieser Pulver aber geringer als die von Zirkonoxidpulvern, so dass der Abrieb an den Gleitflächen reduziert ist.

Es kann erfindungsgemäß auch vorgesehen sein, dass die freie Oberfläche des ersten Knochenzements mit zumindest einem Antibiotikum beschichtet ist.

Besonders bevorzugt ist vorgesehen, dass der zweiteilige Gelenkspacer ein artikulierender Kniespacer ist.

Die Aufgaben der Erfindung werden auch gelöst durch ein Set zum Aufbau eines solchen artikulierenden Spacers aufweisend eine Kartusche und/oder ein Applikationssystem enthaltend den pastösen abriebarmen ersten Knochenzement oder aufweisend ein zweites Kartuschensystem und/oder zweites Mischsystem enthaltend die Ausgangskomponenten für den ersten abriebarmen Knochenzement, aufweisend eine zweite Kartusche und/oder ein zweites Applikationssystem enthaltend den zweiten, antibiotikahaltigen Knochenzement oder aufweisend ein Kartuschensystem und/oder Mischsystem enthaltend die Ausgangskomponenten für den zweiten, antibiotikahaltigen Knochenzement, und aufweisend wenigstens zwei Spacerformen zur Herstellung eines Formteils aus dem ersten abriebarmen Knochenzement, wobei die inneren Oberflächen der Spacerformen ein Negativ der zu erzeugenden Gleitflächen umfassen.

Ebenso können die Aufgaben der Erfindung gelöst werden durch ein Set zum Aufbau eines solchen artikulierenden Spacers, aufweisend zumindest zwei Spacerbauteile, die aus dem ersten Knochenzement bestehen und die jeweils eine Gleitfläche des Spacers umfassen, und aufweisend eine Kartusche und/oder ein Applikationssystem enthaltend den zweiten, antibiotikahaltigen Knochenzement oder aufweisend ein Kartuschensystem und/oder Mischsystem enthaltend die Ausgangskomponenten für den zweiten, antibiotikahaltigen Knochenzement.

Solche Sets sind einfach anwendbar und bieten dem Anwender aber gleichzeitig die Möglichkeit zu einer individuellen Behandlung des Patienten. Beispielsweise kann eine individuelle und besonders geeignete Antibiotika-Mischung für den zweiten Knochenzement eingesetzt werden. Auch ist es vorstellbar, dass eine besonders gut passende Spacerform aus einer Mehrzahl von verschiedenen Spacerformen und Spacergrößen ausgewählt wird, die besonders gut zur Anatomie des Patienten oder zur Behandlungssituation, insbesondere zur individuellen Anpassung der Infektionsbehandlung (Grad der Debridement erst während des Operationsvorgang sichtbar) passt.

Die Aufgaben der Erfindung werden ferner gelöst durch ein Verfahren zur Herstellung eines zweiteiligen artikulierenden Gelenkspacers, bei dem bei beiden Spacerteilen eine Gleitfläche aus einem abriebarmen ersten Knochenzement ausgeformt wird und zumindest die Oberflächen der restlichen Spacerteile zu zumindest 50% mit einem im Vergleich zum ersten Knochenzement unterschiedlichen zweiten Knochenzement ausgeformt werden, der wenigstens ein wasserlösliches Antibiotikum enthält, wobei der erste Knochenzement im Bereich der Gleitflächen mit einer Dicke von zumindest 1 mm auf dem zweiten Knochenzement angeordnet wird.

Bevorzugt kann dabei vorgesehen sein, dass der zweite Knochenzement mit einem Antibiotikum oder einer Mischung verschiedener Antibiotika hergestellt wird, bevorzugt ausgewählt aus Gentamicin, Vancomycin und Clindamycin.

Solche Mischungen sind besonders gut zur Behandlung der Infektionen am Gelenk geeignet.

Mit einer Weiterentwicklung des erfindungsgemäßen Verfahrens wird vorgeschlagen, dass in den ersten Knochenzement ein röntgenopakes Pulver mit einer Mohshärte von weniger als 8 eingemischt wird, bevorzugt eine Mohshärte von weniger als 6, besonders bevorzugt eine Mohshärte von weniger als 4 eingemischt wird, insbesondere Calciumcarbonatpulver und/oder Bariumcarbonatpulver.

Je geringer die Härte des röntgenopaken Pulvers des ersten Knochenzements ist, desto weniger tragen diese zum Abreiben der Gleitflächen bei.

Mit einer bevorzugten Weiterentwicklung des Verfahrens wird vorgeschlagen, dass zuerst der zweite Knochenzement ausgeformt wird und anschließend der erste Knochenzement auf den zweiten Knochenzement aufgebracht wird und die Gleitflächen ausgeformt werden.

Alternativ kann vorgesehen sein, dass zuerst der erste Knochenzement in einer Spacerform gefüllt wird, wobei die Gleitfläche durch eine negative Form der Innenseite der Spacerform geformt wird und anschließend der zweite Knochenzement auf den ersten Knochenzement aufgebracht wird und restliche Oberfläche der Spacerteile ausgeformt wird. Dabei kann erfindungsgemäß bevorzugt vorgesehen sein, dass die restliche Oberfläche der Spacerteile über eine negative Form der Spacerform ausgeformt wird. Alternativ oder auch zusätzlich kann ferner bevorzugt vorgesehen sein, dass die ausgehärteten Spacerteile mit dem zweiten Knochenzement an den Knochen befestigt werden.

Diese Verfahren eignen sich im oft hektischen Betrieb eines OPs zur einfachen Umsetzung.

Der Erfindung liegt die überraschende Erkenntnis zugrunde, dass es durch die Verwendung zweier Knochenzemente gelingt, eine stabilere Lauffläche beziehungsweise Gleitfläche der artikulierenden Spacerteile zu erzeugen, ohne dass die Vorteile eines Antibiotika freisetzenden Spacers verloren gehen. Der so erzeugte Spacer führt zu weniger Problemen während er eingesetzt ist.

Im Folgenden werden Ausführungsbeispiele der Erfindung anhand von einer schematisch dargestellten Figur erläutert, ohne jedoch dabei die Erfindung zu beschränken.

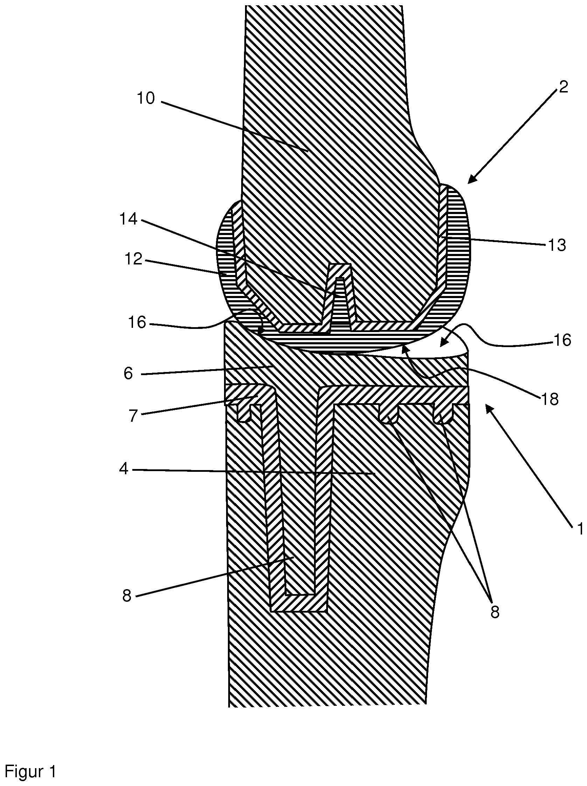

Figur 1 zeigt eine schematische Querschnittansicht eines erfindungsgemäßen Spacers, der aus zwei Knochenzementen (6, 7, 12, 13) gefertigt ist. Der gezeigte Spacer ist ein zweiteiliger artikulierender Kniespacer der zum temporären Ersetzen eines Kniegelenks vorgesehen ist. Als dargestellte Schnittebene ist eine zur Sagitalebene parallele Ebene des künstlichen Kniegelenks gewählt.

Die zwei Teile des Kniespacers sind ein Tibiateil 1 (in Figur 1 unten) und ein Femurteil 2 (In Figur 1 oben). Der Tibiateil 1 ist an einen Tibiaknochen 4 eines Patienten befestigt. Der Tibiateil 1 weist einen Gelenkkopf mit einer Gelenkpfanne auf. Der Gelenkkopf ist aus einem ersten Knochenzement 6 gefertigt, der über eine zur Vermeidung eines Abriebs ausreichende Härte durch Verwendung von Bariumcarbonatpulver und/oder Calciumcarbonatpulver als Röntgenopaker verfügt und - optional - Antibiotika enthalten kann / Antibiotika-haltig sein kann. Der erste Knochenzement 6 ist dadurch abriebarm. Der restliche Tibiateil 1 ist aus einem zweiten Knochenzement 7 gefertigt, in dem eine Mischung zweier in Wasser lösbarer Antibiotika enthalten ist, die auf die Behandlungssituation des Patienten abgestimmt sind.

Der Gelenkkopf ist über einen konischen Zapfen 8 in dem Tibiaknochen 4 verankert. Dadurch wird eine stabile Verbindung des Tibiateils 1 mit dem Knochen 4 erreicht und es wird die Menge der verwendeten Antibiotika haltigen zweiten Knochenzements 7 verringert und damit Antibiotika eingespart. Daneben werden noch weitere Verankerungen 8 aus dem zweiten Knochenzement 7 in passenden konischen Ausnehmungen im Tibiaknochen 4 verankert, um eine stabilere Verbindung des Tibiateils 1 des Spacers mit dem Tibiaknochen 4 zu erreichen.

Der Femurteil 2 des Spacers ist in gleicher Weise aufgebaut. Auf dem Femurknochen 10 eines Patienten, ist aus einem abriebarmen Knochenzement 12 ein Gelenkkopf geformt und mit einem antibiotikahaltigen zweiten Knochenzement 13 befestigt. Der erste Knochenzement 12 des Femurteils 2 des Spacers ist bevorzugt der gleiche wie der erste Knochenzement 6 des Tibiateils 1 des Spacers und der zweite Knochenzement 13 des Femurteils 2 des Spacers ist bevorzugt der gleiche wie der zweite Knochenzement 7 des Tibiateils 1 des Spacers. Eine stabile Verbindung des Gelenkkopfs mit dem Femurknochen 10 wird mit Hilfe einer konischen Verankerung 14 erreicht.

Bis auf die Gelenkköpfe sind die Oberflächen der beiden Spacerteile 1, 2 durch den zweiten Knochenzement 7, 13 realisiert und können daher im beim Patienten eingesetzten Zustand Antibiotika abgeben, insbesondere in Richtung der Knochen 4, 10 des Patienten.

Die beiden Spacerteile 1, 2 liegen in dem vorgesehen Zustand, das heißt, in dem beim Patienten eingesetzten Zustand, wie in Figur 1 gezeigt, über die Gelenkköpfe aneinander an. Dazu sind an den Oberflächen der Gelenkköpfe ähnlich einem natürlichen Kniegelenk Gleitflächen 16, 18 vorgesehen, über die die beiden Spacerteile 1, 2 aufeinander abrollen beziehungsweise übereinander gleiten können. Dadurch ist eine Artikulation der Spacerteile 1, 2 und damit die Nachbildung der Funktionsweise des Knies möglich.

Da die Gleitflächen 16, 18 aus dem ersten, abriebarmen Knochenzement 6, 12 gefertigt sind, bleiben die Gleitflächen 16, 18 während der Verweildauer des temporären Kniespacers intakt, so dass sich keine (oder nur sehr wenige) Partikel von den Gleitflächen 16, 18 ablösen. Dadurch bleibt die Beweglichkeit des Gelenkspacers erhalten und es erfolgt keine Beeinträchtigung der Heilung durch den Abrieb.

Gleichzeitig wird zur Unterstützung der Heilung die Antibiotikamischung aus dem zweiten Knochenzement 7, 13 der beiden Spacerteile 1, 2 fortwährend herausgelöst und steht zur Bekämpfung von Infektionen zur Verfügung.

Das gezeigte Beispiel bezieht sich auf einen erfindungsgemäß bevorzugten Kniespacer. Die Erfindung ist jedoch nicht auf Kniespacer beschränkt, sondern betrifft auch alle anderen Formen von zweiteiligen temporären Gelenkspacern, wie beispielsweise Ellenbogenspacer, Hüftgelenkspacer, Fußgelenkspacer oder Schultergelenkspacer. Für den Fachmann ist das mit Figur 1 beschriebene Beispiel eines Spacers für ein Knie ohne weiteres auf Spacer für andere Gelenke übertragbar.

Die in der voranstehenden Beschreibung, sowie den Ansprüchen, Figuren und Ausführungsbeispielen offenbarten Merkmale der Erfindung können sowohl einzeln, als auch in jeder beliebigen Kombination für die Verwirklichung der Erfindung in ihren verschiedenen Ausführungsformen wesentlich sein, wobei der Schutzbereich der Erfindung einzig durch die Ansprüche definiert wird.

### Bezugszeichenliste

- 1: Spacerteil / Tibiateil
- 2: Spacerteil / Femurteil
- 4: Knochen (Tibia)
- 6: Abriebarmer Knochenzement (Tibiateil)
- 7: Antibiotikahaltiger Zement (Tibiateil)
- 8: Verankerung
- 10: Knochen (Femur)
- 12: Abriebarmer Knochenzement (Femurteil)
- 13: Antibiotikahaltiger Zement (Femurteil)
- 14: Verankerung
- 16: Gleitfläche (Tibiateil)
- 18: Gleitfläche (Femurteil)

## Patentansprüche

1. Artikulierender Gelenkspacer zum temporären Ersetzen eines Gelenks, wobei der Gelenkspacer zwei Spacerteile (1, 2) aufweist, die jeweils eine Gleitfläche (16, 18) aufweisen, auf der die Spacerteile (1, 2) im beim Patienten eingesetzten Zustand beweglich aneinander anliegen und aufeinander abrollen, **dadurch gekennzeichnet, dass** die Gleitflächen (16, 18) durch einen abriebarmen ersten Knochenzement (6, 12) gebildet sind und die restlichen Spacerteile (1, 2) zumindest teilweise aus einem im Vergleich zum ersten Knochenzement unterschiedlichen zweiten Knochenzement (7, 13) gebildet sind, der wenigstens ein in Wasser lösliches Antibiotikum enthält, wobei der erste Knochenzement (6, 12) im Bereich der Gleitflächen (16, 18) mit einer Dicke von zumindest 1 mm auf dem zweiten Knochenzement (7, 13) angeordnet ist.

2. Spacer nach Anspruch 1, **dadurch gekennzeichnet, dass**
der erste Knochenzement (6, 12) im Bereich der Gleitflächen (16, 18) mit einer Dicke zwischen 2 mm und 15 mm angeordnet ist, besonders bevorzugt mit einer Dicke zwischen 6 mm und 11 mm angeordnet ist.

3. Spacer nach Anspruch 1 oder 2, **dadurch gekennzeichnet, dass**
der erste Knochenzement (6, 12) zumindest eine Verankerung (8, 14) aufweist, die sich aus Richtung der Gleitfläche (16, 18) konisch in die restlichen Spacerteile (1, 2) fortsetzt, insbesondere in den zweiten Knochenzement (7, 13) fortsetzt.

4. Spacer nach einem der vorangehenden Ansprüche, **dadurch gekennzeichnet, dass** die restlichen Spacerteile (1, 2) vollständig aus dem zweiten Knochenzement (7, 13) bestehen und zumindest die Verbindungen des Gelenkspacers zum Knochen (4, 10) des Patienten aus dem zweiten Knochenzement (7, 13) bestehen, bevorzugt auch die von der Gleitfläche (16, 18) beabstandeten Bereiche der Gelenkköpfe (6, 12) des Gelenkspacers aus dem zweiten Knochenzement (7, 13) bestehen, besonders bevorzugt die von der Gleitfläche (16, 18) zumindest 1 mm beabstandeten Bereiche der Gelenkköpfe (6, 12) des Gelenkspacers aus dem zweiten Knochenzement (7, 13) bestehen.

5. Spacer nach einem der vorangehenden Ansprüche, **dadurch gekennzeichnet, dass** der abriebarme erste Knochenzement (6, 12) ein röntgenopakes Pulver mit einer Mohshärte von weniger als 8 beinhaltet, bevorzugt eine Mohshärte von weniger als 6, besonders bevorzugt eine Mohshärte von weniger als 4 beinhaltet.

6. Spacer nach einem der vorangehenden Ansprüche, **dadurch gekennzeichnet, dass** der zweite Knochenzement (7, 13) eine Mischung aus zumindest zwei Antibiotika enthält, bevorzugt ausgewählt aus Gentamicin, Vancomycin und Clindamycin.

7. Spacer nach einem der vorangehenden Ansprüche, **dadurch gekennzeichnet, dass** die freie Oberfläche des ersten Knochenzements mit zumindest einem Antibiotikum beschichtet ist.

8. Spacer nach einem der vorangehenden Ansprüche, **dadurch gekennzeichnet, dass** der abriebarme erste Knochenzement (6, 12) ein Calciumcarbonatpulver und/oder Bariumcarbonatpulver beinhaltet.

9. Set zum Aufbau eines artikulierenden Spacers nach einem der vorangehenden Ansprüche,
aufweisend eine Kartusche und/oder ein Applikationssystem enthaltend den pastösen abriebarmen ersten Knochenzement (6, 12) oder aufweisend ein zweites Kartuschensystem und/oder zweites Mischsystem enthaltend die Ausgangskomponenten für den ersten abriebarmen Knochenzement (6, 12), aufweisend eine zweite Kartusche und/oder ein zweites Applikationssystem enthaltend den zweiten, antibiotikahaltigen Knochenzement (7, 13) oder aufweisend ein Kartuschensystem und/oder Mischsystem enthaltend die Ausgangskomponenten für den zweiten, antibiotikahaltigen Knochenzement (7, 13), und
wenigstens zwei Spacerformen zur Herstellung eines Formteils aus dem ersten abriebarmen Knochenzement (6, 12), wobei die inneren Oberflächen der Spacerformen ein Negativ der zu erzeugenden Gleitflächen (16, 18) umfassen.

10. Set zum Aufbau eines artikulierenden Spacers nach einem der Ansprüche 1 bis 8, aufweisend zumindest zwei Spacerbauteile, die aus dem ersten Knochenzement (6, 12) bestehen und die jeweils eine Gleitfläche (16, 18) des Spacers umfassen, und aufweisend eine Kartusche und/oder ein Applikationssystem enthaltend den zweiten, antibiotikahaltigen Knochenzement (7, 13) oder aufweisend ein Kartuschensystem und/oder Mischsystem enthaltend die Ausgangskomponenten für den zweiten, antibiotikahaltigen Knochenzement (7, 13).

11. Verfahren zur Herstellung eines zweiteiligen artikulierenden Gelenkspacers, bei dem bei beiden Spacerteilen (1, 2) eine Gleitfläche (16, 18) aus einem abriebarmen ersten Knochenzement (6, 12) ausgeformt wird und zumindest die Oberflächen der restlichen Spacerteile (1, 2) zu zumindest 50% mit einem im Vergleich zum ersten Knochenzement unterschiedlichen zweiten Knochenzement (7, 13) ausgeformt werden, der wenigstens ein wasserlösliches Antibiotikum enthält, wobei der erste Knochenzement (6, 12) im Bereich der Gleitflächen (16, 18) mit einer Dicke von zumindest 1 mm auf dem zweiten Knochenzement (7, 13) angeordnet wird.

12. Verfahren nach Anspruch 11, **dadurch gekennzeichnet, dass**
der zweite Knochenzement (7, 13) mit einem Antibiotikum oder einer Mischung verschiedener Antibiotika hergestellt wird, bevorzugt ausgewählt aus Gentamicin, Vancomycin und Clindamycin.

13. Verfahren nach Anspruch 11 oder 12, **dadurch gekennzeichnet, dass** in den ersten Knochenzement (6, 12) ein röntgenopakes Pulver mit einer Mohshärte von weniger als 8 eingemischt wird, bevorzugt eine Mohshärte von weniger als 6, besonders bevorzugt eine Mohshärte von weniger als 4 eingemischt wird, insbesondere Calciumcarbonatpulver und/oder Bariumcarbonatpulver.

14. Verfahren nach einem der Ansprüche 11 bis 13, **dadurch gekennzeichnet, dass** zuerst der zweite Knochenzement (7, 13) ausgeformt wird und anschließend der erste Knochenzement (6, 12) auf den zweiten Knochenzement (7, 13) aufgebracht wird und die Gleitflächen (16, 18) ausgeformt werden.

15. Verfahren nach einem der Ansprüche 11 bis 13, **dadurch gekennzeichnet, dass** zuerst der erste Knochenzement (6, 12) in eine Spacerform gefüllt wird, wobei die Gleitfläche (16, 18) durch eine negative Form der Innenseite der Spacerform geformt wird und anschließend der zweite Knochenzement auf den ersten Knochenzement aufgebracht wird und die restliche Oberfläche der Spacerteile (1, 2) ausgeformt wird, bevorzugt über eine negative Form der Spacerform ausgeformt wird und/oder die ausgehärteten Spacerteile (1, 2) mit dem zweiten Knochenzement (7, 13) an den Knochen (4, 10) befestigt werden.

## Claims

1. Articulating joint spacer for temporary replacement of a joint, whereby the joint spacer comprises two spacer parts (1, 2), which each comprise one sliding surface (16, 18) on which the spacer parts (1, 2), in the patient-inserted state, touch against each other in mobile manner and roll off on each other **characterised in that**
the sliding surfaces (16, 18) are made of a low-abrasion first bone cement (6, 12) and the remaining spacer parts (1, 2) are made, at least in part, of a second bone cement (7, 13) being different to the first bone cement, the second bone cement containing at least one water-soluble antibiotic, whereby the first bone cement (6, 12) is arranged on the second bone cement (7, 13) in the region of the sliding surfaces (16, 18) with a thickness of at least 1 mm.

2. Spacer according to claim 1, **characterised in that**
the first bone cement (6, 12) is arranged on the second bone cement (7, 13) in the region of the sliding surfaces (16, 18) with a thickness between 2 mm and 15 mm, particularly preferably is arranged with a thickness between 6 and 11 mm.

3. Spacer according to claim 1 or 2, **characterised in that**
the first bone cement (6, 12) comprises at least one anchoring (8, 14) that extends from the direction of the sliding surface (16, 18) conically into the remaining spacer parts (1, 2), in particular into the second bone cement (7, 13).

4. Spacer according to any one of the preceding claims, **characterised in that** the remaining spacer parts (1, 2) fully consist of the second bone cement (7, 13) and at least the connections of the joint spacer to the bone (4, 10) of the patient consist of the second bone cement (7, 13), preferably the regions of the joint heads (6, 12) of the joint spacer situated at a distance from the sliding surface (16, 18) also consist of the second bone cement (7, 13), particularly preferably the regions of the joint heads (6, 12) of the joint spacer situated at a distance of at least 1 mm from the sliding surface (16, 18) consist of the second bone cement (7, 13).

5. Spacer according to any one of the preceding claims, **characterised in that** the low-abrasion first bone cement (6, 12) contains a radiopaque powder with a Mohs hardness of less than 8, preferably a Mohs hardness of less than 6, particularly preferably a Mohs hardness of less than 4.

6. Spacer according to any one of the preceding claims, **characterised in that** the second bone cement (7, 13) contains a mixture of at least two antibiotics, preferably selected from gentamicin, vancomycin, and clindamycin.

7. Spacer according to any one of the preceding claims, **characterised in that** the free surface of the first bone cement is coated by at least one antibiotic.

8. Spacer according to any one of the preceding claims, **characterised in that** the low-abrasion first bone cement (6, 12) contains a calcium carbonate powder and/or barium carbonate powder.

9. Set for build-up of an articulating spacer according to any one of the preceding claims, comprising a cartridge and/or an application system containing the pasty low-abrasion first bone cement (6, 12), or comprising a second cartridge system and/or second mixing system containing the starting components for the low-abrasion first bone cement (6, 12),
comprising a second cartridge and/or a second application system containing the antibiotic-containing second bone cement (7, 13), or comprising a cartridge system and/or mixing system containing the starting components for the antibiotic-containing second bone cement (7, 13), and
at least two spacer moulds for producing a moulded part from the low-abrasion first bone cement (6, 12), whereby the internal surfaces of the spacer moulds comprise a negative image of the sliding surfaces (16, 18) to be produced.

10. Set for build-up of an articulating spacer according to any one of the claims 1 to 8, comprising at least two spacer components that consist of the first bone cement (6, 12) and each comprise one sliding surface (16, 18) of the spacer, and
comprising a cartridge and/or an application system containing the antibiotic-containing second bone cement (7, 13) or comprising a cartridge system and/or mixing system containing the starting components for the antibiotic-containing second bone cement (7, 13).

11. Method for producing a two-part articulating joint spacer, in which a sliding surface (16, 18) is formed in both spacer parts (1, 2) from a low-abrasion first bone cement (6, 12) and at least the surfaces of the remaining spacer parts (1, 2) are formed with a second bone cement (7, 13) being different to the first bone cement, at least at a level of 50%, which contains at least one water-soluble antibiotic, whereby the first bone cement (6, 12) is arranged on the second bone cement (7, 13) in the region of the sliding surfaces (16, 18) with a thickness of at least 1 mm.

12. Method according to claim 11, **characterised in that**
the second bone cement (7, 13) is produced such as to contain an antibiotic or a mixture of different antibiotics, preferably selected from gentamicin, vancomycin, and clindamycin.

13. Method according to claim 11 or 12, **characterised in that** a radiopaque powder with a Mohs hardness of less than 8, preferably with a Mohs hardness of less than 6, particularly preferably with a Mohs hardness of less than 4, in particular calcium carbonate powder and/or barium carbonate powder, is admixed to the first bone cement (6, 12).

14. Method according to any one of the claims 11 to 13, **characterised in that** the second bone cement (7, 13) is formed first and then the first bone cement (6, 12) is applied onto the second bone cement (7, 13) and the sliding surfaces (16, 18) are formed.

15. Method according to any one of the claims 11 to 13, **characterised in that** the first bone cement (6, 12) is filled into a spacer mould first, whereby the sliding surface (16, 18) is formed by a negative image of the inside of the spacer mould, and then the second bone cement is applied onto the first bone cement and the remaining surface of the spacer parts (1, 2) is formed, preferably is formed through a negative mould of the spacer mould and/or the cured spacer parts (1, 2) are attached to the bone (4, 10) by means of the second bone cement (7, 13).

## Revendications

1. Espaceur d'articulation destiné au remplacement temporaire d'une articulation, où l'espaceur d'articulation présente deux parties d'espaceur (1, 2), qui présentent chacune une surface de glissement (16, 18) sur laquelle les parties d'espaceur (1, 2) sont ajustées pour se mouvoir l'une par rapport à l'autre et roulent l'une sur l'autre à l'état implanté chez le patient, **caractérisé en ce que**
les surfaces de glissement (16, 18) sont formées par un premier ciment osseux (6, 12) de faible abrasion et les parties d'espaceur (1, 2) restantes sont formées au moins partiellement à partir d'un deuxième ciment osseux (7, 13), différent en comparaison avec le premier ciment osseux, qui contient au moins un antibiotique soluble dans l'eau, où le premier ciment osseux (6, 12) est disposé dans la région des surfaces de glissement (16, 18) avec une épaisseur d'au moins 1 mm sur le deuxième ciment osseux (7, 13).

2. Espaceur selon la revendication 1, **caractérisé en ce que**
le premier ciment osseux (6, 12) est disposé dans la région des surfaces de glissement (16, 18) avec une épaisseur entre 2 mm et 15 mm, de manière particulièrement préférée, avec une épaisseur entre 6 mm et 11 mm.

3. Espaceur selon la revendication 1 ou 2, **caractérisé en ce que**
le premier ciment osseux (6, 12) présente au moins un ancrage (8, 14) qui se poursuit de manière conique dans les parties d'espaceurs (1, 2) restantes à partir de la direction des surfaces de glissement (16, 18), notamment, se poursuit dans le deuxième ciment osseux (7, 13).

4. Espaceur selon l'une des revendications précédentes, **caractérisé en ce que**
les parties d'espaceur (1, 2) restantes sont constituées totalement du deuxième ciment osseux (7, 13) et qu'au moins les liaisons de l'espaceur d'articulation vers l'os (4, 10) du patient sont constituées du deuxième ciment osseux (7, 13), de préférence également les régions des têtes d'articulations (6, 12) de l'espaceur d'articulation espacées par rapport à la surface de glissement (16, 18) sont constituées du deuxième ciment osseux (7, 13), de manière particulièrement préférée, les régions des têtes d'articulation (6, 12) de l'espaceur d'articulation espacées d'au moins 1 mm par rapport à la surface de glissement (16, 18) sont constituées du deuxième ciment osseux (7, 13).

5. Espaceur selon l'une des revendications précédentes, **caractérisé en ce que** le premier ciment osseux (6, 12) de faible abrasion contient une poudre opaque aux rayons X avec une dureté de Mohs inférieure à 8, de préférence une dureté de Mohs inférieure à 6, de manière particulièrement préférée une dureté de Mohs inférieure à 4.

6. Espaceur selon l'une des revendications précédentes, **caractérisé en ce que** le deuxième ciment osseux (7, 13) contient un mélange d'au moins deux antibiotiques, de préférence choisis parmi la gentamicine, la vancomycine et la clindamycine.

7. Espaceur selon l'une des revendications précédentes, **caractérisé en ce que** la surface supérieure libre du premier ciment osseux est recouverte avec au moins un antibiotique.

8. Espaceur selon l'une des revendications précédentes, **caractérisé en ce que** le premier ciment osseux (6, 12) de faible abrasion (6, 12) contient une poudre à base de carbonate de calcium et/ou une poudre à base de carbonate de baryum.

9. Ensemble pour l'élaboration d'un espaceur articulé selon l'une des revendications précédentes,
présentant une cartouche et/ou un système d'application contenant le premier ciment osseux (6, 12) pâteux de faible abrasion ou présentant un deuxième système de cartouche et/ou un deuxième système de mélange contenant les produits de départ pour le premier ciment osseux (6, 12) de faible abrasion, présentant une deuxième cartouche et/ou un deuxième système d'application contenant le deuxième ciment osseux (7, 13) contenant un antibiotique ou présentant un système de cartouche et/ou un système de mélange contenant les composants de départ pour le deuxième ciment osseux (7, 13) contenant un antibiotique, et
au moins deux moules d'espaceur pour la fabrication d'un moulage à partir du premier ciment osseux (6, 12) de faible abrasion, où les surfaces supérieures intérieures des moules d'espaceur comprennent un négatif des surfaces de glissement (16, 18) à créer.

10. Ensemble pour l'élaboration d'un espaceur articulant selon l'une des revendications 1 à 8,
présentant au moins deux composants d'espaceur qui sont constitués du premier ciment osseux (6, 12) et qui comprennent chacun une surface de glissement (16, 18) de l'espaceur, et
présentant une cartouche et/ou un système d'application contenant le deuxième ciment osseux (7, 13) contenant un antibiotique ou présentant un système de cartouche et/ou un système de mélange contenant les composants de départ pour le deuxième ciment osseux (7, 13) contenant un antibiotique.

11. Procédé de fabrication d'un espaceur d'articulation en deux parties, chez lequel, pour les deux parties d'espaceur, on moule une surface de glissement (16, 18) à partir d'un premier ciment osseux (6, 12) de faible abrasion et au moins les surfaces supérieures des parties d'espaceur (1, 2) restantes sont façonnées à au moins 50 % avec un deuxième ciment osseux (7, 13) différent en comparaison du premier ciment osseux, qui contient au moins un antibiotique soluble dans l'eau, où le premier ciment osseux (6, 12) est disposé dans la région des surfaces de glissement (16, 18) avec une épaisseur d'au moins 1 mm sur le deuxième ciment osseux (7, 13).

12. Procédé selon la revendication 11, **caractérisé en ce que**
le deuxième ciment osseux (7, 13) est fabriqué avec un antibiotique ou un mélange de divers antibiotiques, de préférence, choisis parmi la gentamicine, la vancomycine et la clindamycine.

13. Procédé selon la revendication 11 ou 12, **caractérisé en ce**
**qu'**une poudre opaque aux rayons X avec une dureté de Mohs inférieure à 8, de préférence une dureté de Mohs inférieure à 6, de manière particulièrement préférée une dureté de Mohs inférieure à 4, notamment une poudre à base de carbonate de calcium et/ou une poudre à base de carbonate de baryum, est incorporée dans le premier ciment osseux (6, 12).

14. Procédé selon l'une des revendications 11 à 13, **caractérisé en ce que**
le deuxième ciment osseux (7, 13) est d'abord moulé et ensuite le premier ciment osseux (6, 12) est rapporté sur le deuxième ciment osseux (7, 13) et les surfaces de glissement (16, 18) sont moulées.

15. Procédé selon l'une des revendications 11 à 13, **caractérisé en ce que**
le premier ciment osseux (6, 12) est déposé d'abord dans un moule d'espaceur, où la surface de glissement (16, 18) est moulée par une forme négative de la face intérieure du moule d'espaceur et ensuite le deuxième ciment osseux est rapporté sur le premier ciment osseux, et
la surface supérieure restante des parties d'espaceur (1, 2) est moulée, de préférence, par l'intermédiaire d'une forme négative du moule et/ou les parties d'espaceur (1, 2) durcies sont fixées sur l'os (4, 10) avec le deuxième ciment osseux (7,13).
